# EUROPEAN PATENT SPECIFICATION

(11) **EP 3 058 903 B1**
(45) Date of publication and mention of the grant of the patent: **19.12.2018**
(21) Application number: 15155760.0
(22) Date of filing: 19.02.2015
(51) Int. Cl.: A61F 5/01, A61F 5/14

(54) **Support apparatus with adjustable stiffness**
Trägervorrichtung mit einstellbarer Steifigkeit
Appareil de support à rigidité réglable

(43) Date of publication of application: 24.08.2016
(73) Proprietor: Podfo Limited, Long Benton Newcastle upon Tyne NE12 8BW (GB)
(72) Inventor: PALLARI, Jari, Newcastle upon Tyne Tyne and Wear NE7 7QJ (GB); COOK, Stephen James, Fovant Wiltshire SP3 5LF (GB)
(74) Representative: Vinsome, Rex Martin

(56) References cited:
- EP-A2- 2 034 932
- US-A1- 2002 014 024
- US-A1- 2005 235 526
- US-A1- 2006 276 735
- US-A1- 2010 275 469
- US-A1- 2013 178 344

## Description

This invention relates to a support apparatus having adjustable stiffness for providing tailored support to a body part of a user, and relates particularly, but not exclusively, to an orthotic and/or prosthetic apparatus.

Orthotic devices, or orthoses, are medical devices used to treat or prevent a number of patient pathologies or musculoskeletal problems, or to enhance a user's performance. For example, conditions such as plantar fasciitis, pressure lesions on the feet, pain from arthritis, and conditions in other parts of the body that require the modification of kinetics in the lower limbs and feet, can be treated with foot orthoses.

A simple foot orthotic comprises a form-fitting insole that usually conforms to the shape of a user's foot on the upper side and can have a generic shoe-fitting shape on the lower side. In some cases, the foot orthotic is a shell of largely uniform thickness with a heel element added.

Foot orthotics work by altering the kinematics of the forces experienced by the user's foot in a desired way and can consequently reduce forces acting in another part of the body during the gait cycle. They can also be used to redistribute the pressure on the foot to reduce pathological forces or alter the centre of mass of the user.

Foot orthotics utilise a range of materials to treat patient pathologies. These materials include carbon fibre, ethylene-vinyl acetate, gels, and polypropylene. Most foot orthotics are mass-produced but bespoke foot orthotics are also made to treat a patient in a more focussed and effective way.

3D printing, or additive manufacturing, methods involve laying down successive layers of material to create a three-dimensional object according to the information in a digital file, such as a CAD file. In this way, appropriate modelling software can accept a file containing information from a three-dimensional scan as input, allow a user to virtually manipulate the scanned object, and subsequently print the object as a new three-dimensional body.

An example of the use of additive manufacturing is the creation of foot orthotics. A three-dimensional scan of a user's foot is uploaded to a computer and a negative cast of the foot sole is designed in software. In combination with data such as the person's gait, or medical or athletic requirements, this negative cast is shaped as required into a bespoke foot orthotic device able to be printed by a 3D printer.

In order to meet the needs of the user of an orthotic device, the device must respond in a desired mechanical way to the user's motion. To do this, the device is required to have a particular shape and stiffness. A device which is not bespoke will not be shaped specifically to a body part of the user and as such cannot provide a tailored mechanical response to the user. If an orthotic device has a largely uniform thickness, its stiffness will also be largely uniform, meaning the device will be unable to provide areas of localised support to the body part of the user.

The needs of the user of an orthotic device change with time. As the user's body changes, such as in response to use of an orthotic device, the user may require a new orthotic device with a different shape or different stiffness specifications to best serve his or her needs after a period of time. This would require being measured for and purchasing another device.

Additive manufacturing methods can also be used to create prosthetic apparatus, for example prosthetic sockets such as transtibial/femoral/humeral/radial prosthetic sockets. Prosthetics replace a part of the anatomy, unlike orthoses which support an existing part. Generally speaking the interface between a prosthetic limb and the residual limb is the most difficult part of the prosthetic to make and these are always bespoke as every residual limb is a different shape. This significantly increases the cost and difficulty of manufacture of the prosthetic apparatus.

Preferred embodiments of the present invention seek to overcome one or more of the above disadvantages associated with the prior art.

US 2002/0014024 A1 discloses an insole device configured to fit the profile of the human foot to promote proprioceptive stimulation of the Golgi tendon organ, the midfoot section of the insole device having a domed structure that is presented to the plantar aspect of the foot at a location found to be the anatomical apex of the foot's arch system.

US 2005/0235526 A1 discloses an arch support device for supporting an arch of a user's foot and comprising an arch reinforcement member disposed under an arch member of the device, capable of compressing during deflection of the arch member.

EP2034932 A2 discloses an orthotic comprising a shell layer configured to receive a removable insert that alters an amount of arch support provided by the orthotic.

US 2010/0275469 A1 discloses an insole comprising a reinforcement element on a side of the insole facing away from the foot in the region of an elastically deformable arch, the reinforcement element being made of a different material with a greater stiffness than the material of the insole and having a convex shape.

US 2013/0178344 A1 discloses a resilient rod that provides adjustable directional resistance and may be incorporated into running shoes or orthotic devices.

US 2006/0276735 A1 discloses an orthotic glove having channels into which resilient stays may be placed.

According to a first aspect of the present invention, there is provided a support apparatus comprising the features of claim 1.

These features provide the advantage of making the mechanical properties of the apparatus more adjustable.

The apparatus can also have its stiffness altered in more than one location on the device at any one time, providing the advantage of further increased adjustability of the apparatus.

The apparatus can have its stiffness modified repeatedly by means of re-engaging at least one said stiffness adjusting member to another location on the body at any time, providing the advantage of increased adaptability of the apparatus to the user's changing needs over time.

Said plurality of first engaging members and/or said plurality of second engaging members may comprise a respective plurality of recesses and/or protrusions. In one embodiment, said plurality of first engaging members comprises at least one first recess and at least one first protrusion, and said plurality of second engaging members comprises at least one second recess and at least one second protrusion.

These features provide the advantage of enabling a stiffness adjusting member to be placed at a plurality of locations on the body.

Said body and/or said first engaging members and/or said second engaging members and/or at least one said stiffness adjusting member may be manufactured by additive manufacturing.

This provides the advantage that the body and first engaging members form a unitary body that has greater inherent robustness than a similar body and similar engaging members having been coupled together with an adhesive or other fixing means. It also provides the advantage that the shape of the apparatus and/or the shapes of its components are more accurately reproduced with respect to the user's requirements.

The apparatus may be an orthotic and/or prosthetic apparatus.

According to a second aspect of the present invention, there is provided a method of altering the stiffness of a support apparatus, the method comprising the features of claim 13.

The method may further comprise applying tensile or compressive force to said body prior to mounting at least one said stiffness adjusting member to said body.

This provides the advantage of further increasing the adjustability of the apparatus.

Preferred embodiments of the present invention will now be described, by way of example only and not in any limitative sense, with reference to the accompanying drawings, in which:
Figure 1 shows a foot orthotic of a first embodiment of the present invention;
Figure 2 shows a foot orthotic of a second embodiment of the invention with stiffness adjusting members;
Figures 3-7 show a range of possible arrangements of protrusions and/or recesses on the body of the foot orthotic of Figure 2;
Figure 8 shows an ankle/foot orthotic of a third embodiment of the invention;
Figure 9 shows a spinal brace of a fourth embodiment of the invention;
Figure 10 shows a prosthetic socket of a fifth embodiment of the invention;
Figures 11-16 show a range of possible embodiments of stiffness adjusting members for use with the embodiments of Figures 1 to 10;
Figure 17 shows a ring-shaped stiffness adjusting member for use in a foot orthotic apparatus;
Figure 18 shows the stiffness adjusting member of Figure 17 having been contoured; and
Figure 19 shows a linear stiffness adjusting member.

Orthotic and prosthetic devices are usually manufactured in a very similar way. Initially a plaster impression of the limb or residual limb is taken. This is then filled with plaster or turned into a positive of the limb (i.e. a duplicate). The orientation and/or shape of the limb is then corrected manually to meet a particular clinical requirement. Thermoformable materials are then vacuum formed on the corrected positive to create the body of the orthotic or the prosthetic sockets. Alternatively, a wet lay-up process can be used for composite materials. The device can then be finished with straps, hinges, cushioning pads or other materials and fitted to the patient. Another way of making foot orthoses consists of first designing the orthotic shape via CAD from 3D scanned plaster or foam box impressions and then milling the finished orthotic directly from foam materials, such as EVA foams of different densities. The milled orthotic is then finished manually.

The plaster casting part of the process can be replaced with 3D scanning of the limb (or part of) and designing the device in a CAD environment. Once the correct orthotic or prosthetic socket shape is established, it can be milled from foam materials to create the corrected positive shape and the thermoforming or composite manufacturing process can then be continued. Prosthetic sockets are usually made using thermoplastic materials such as polypropylene or composites, like carbon fibre. For padding orthoses and prosthetic sockets, similar materials are used. These are various kinds of foam such as ethylene-vinyl acetate (EVA) or material supplied under the trade mark Poron (registered trade mark). Also, silicone, leather and different textile materials can be used.

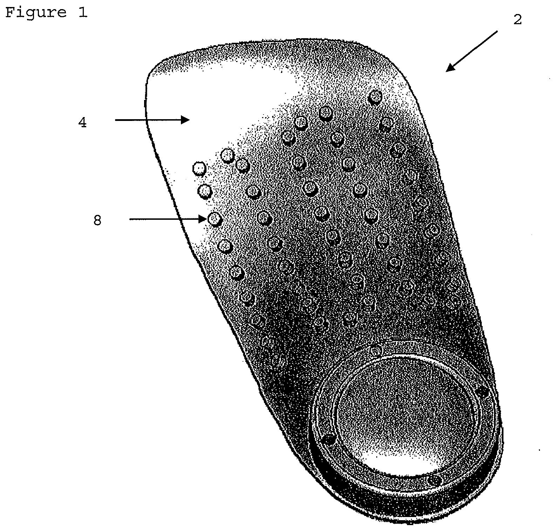

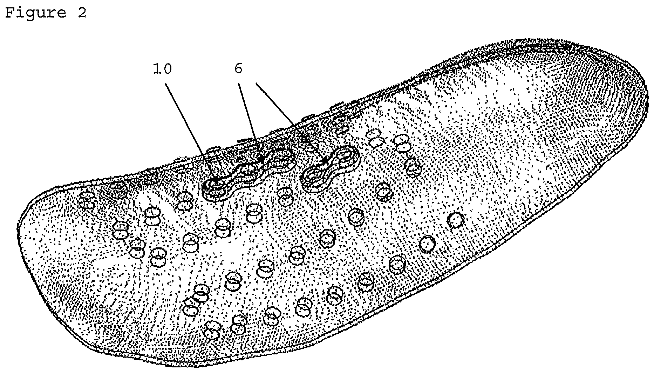

Referring to Figures 1 and 2, the underside of a support apparatus in the form of a foot orthotic 2 embodying the present invention is shown having a body 4 for supporting a foot of a wearer and first engaging means in the form of a plurality of cylindrical, stud-like protrusions 8. These protrusions 8 form first engaging means for the two examples of stiffness adjusting member 6 shown in the form of connectors 6 in Figure 2, whose second engaging means are shown in the form of corresponding recesses 10. It can be seen from Figure 2 that these examples of stiffness adjusting members 6 are designed to connect together two or more protrusions 8, with the effect of increasing the stiffness of the apparatus 2 in the location of the stiffness adjusting member 6.

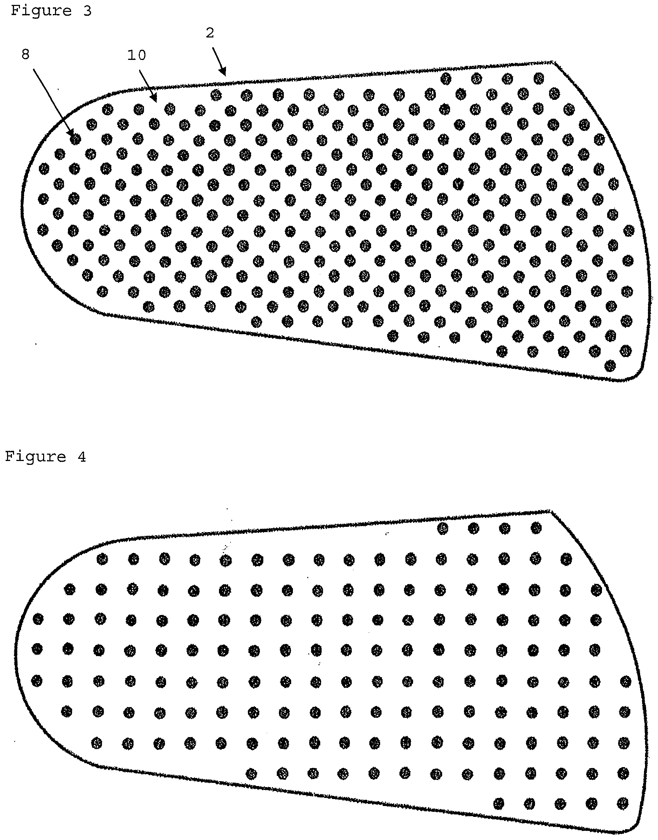

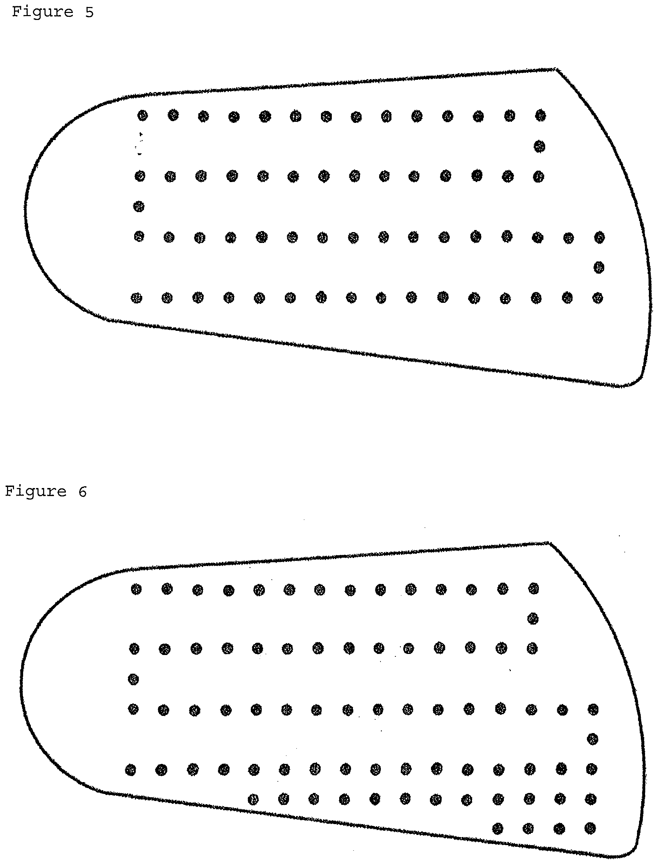

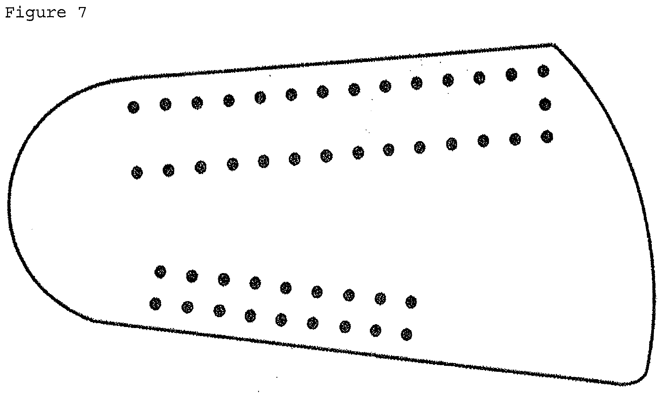

Referring to Figures 3-7, a range of ways in which the protrusions 8 can be arranged on the surface of the foot orthotic 2 are shown. Figure 3 shows a denser arrangement of the protrusions 8, Figures 4 and 5 sparser arrangements, and Figures 6 and 7 show irregular patterns of protrusions 8. The arrangement of protrusions 8 shown in Figure 7, for example, demonstrates an emphasis on the requirement to adjust the stiffness of the foot orthotic 2 in the local regions of the arch and outer edge of a user's foot.

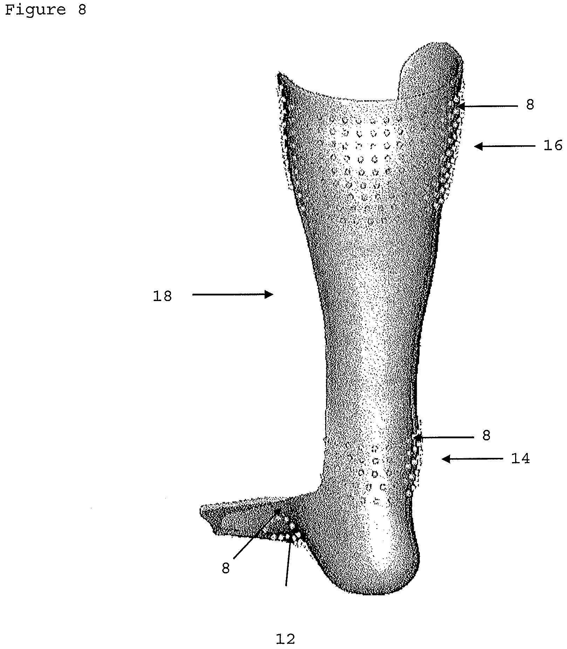

Referring to Figure 8, an ankle/foot orthotic device 18 is shown having an arrangement of protrusions 8 on the outer surface of the device 18 in the regions of the underside of the foot 12, around the ankle region 14, and around the top of the gastrocnemius (calf) muscle region 16.

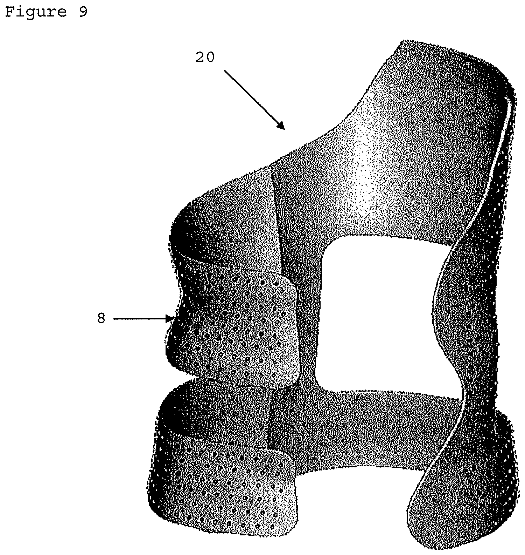

Referring to Figure 9, a spinal brace 20 is shown having an arrangement of protrusions 8 on the outer surface of the brace 20.

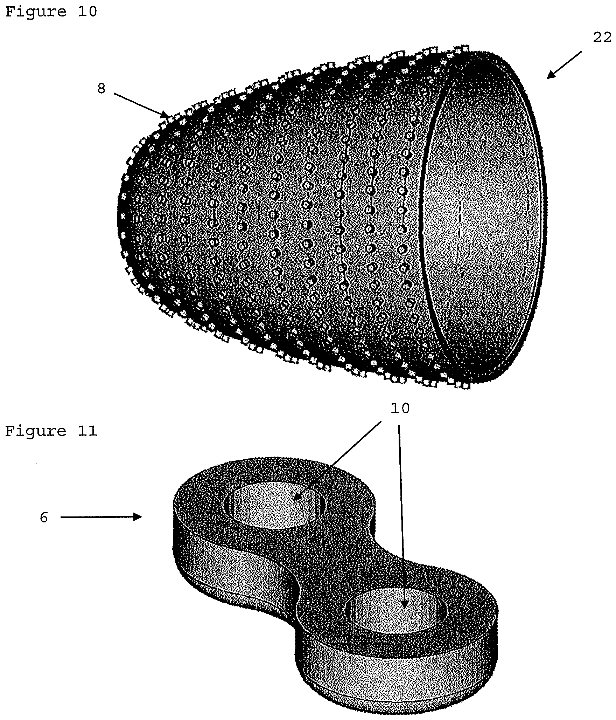

Referring to Figure 10, a prosthetic socket 22 is shown having an arrangement of protrusions 8 on the outer surface of the socket 22.

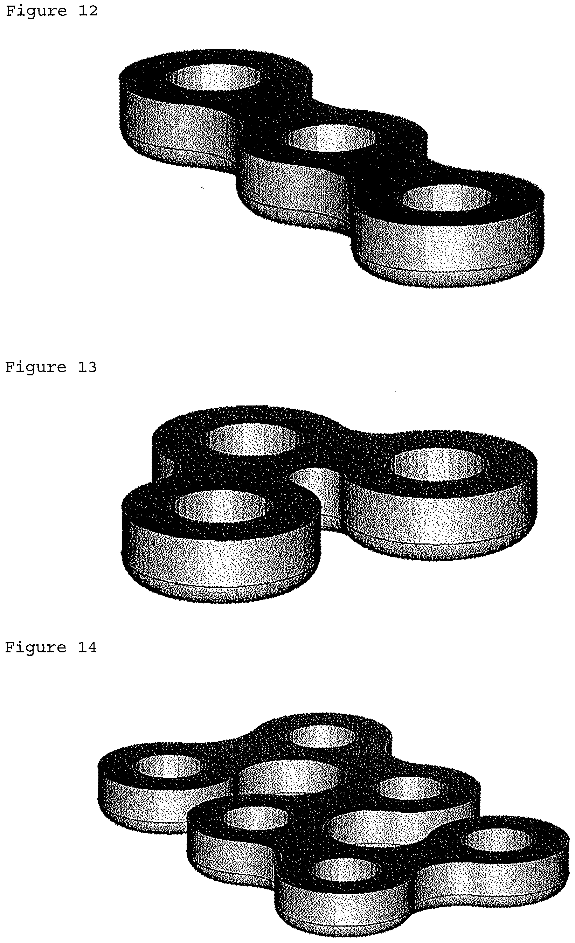

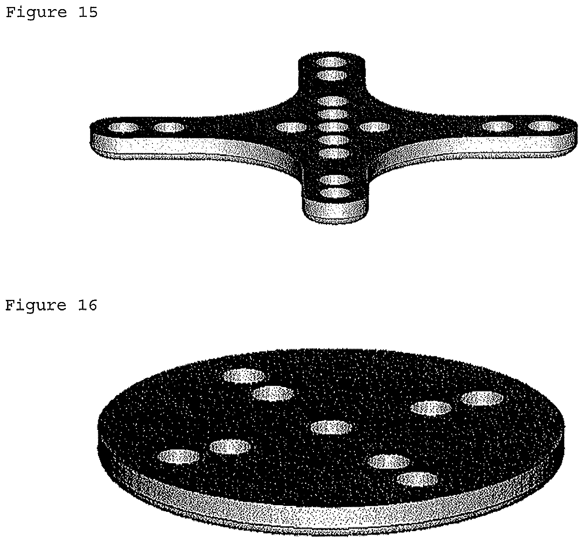

Referring to Figures 11 to 16, examples of stiffness adjusting members 6 are shown wherein the second engaging means takes the form of recesses 10 in the stiffness adjusting members 6.

The shape, size, and material of a stiffness adjusting member 6 can all be chosen prior to use in order to adjust the stiffness of one or more regions of an orthotic device 2. For example, a stiffness adjusting member 6 made from metal in the form of the connector 6 shown in Figure 11 could be chosen to be engaged with protrusions 8 under the arch of a foot orthotic 2, to increase the stiffness in that region to a greater extent than would a connector 6 made from, for example, rubber. In contrast, the same connector 6 may be made from rubber to provide a more yielding adjustment to an orthotic 2 when that adjustment to stiffness is required.

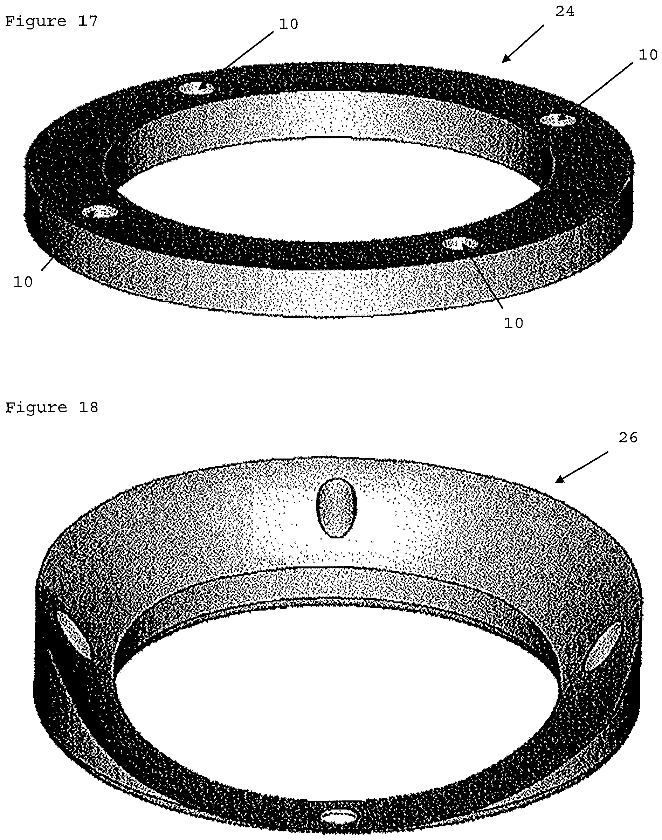

Referring to Figures 17 and 18, a stiffness adjusting member 6 is shown in the form of a circular connector 24 with four recesses 10 as second engaging means. A stiffness adjusting member 6, such as the circular connector 24 of Figure 17, can be contoured like the connector 26 shown in Figure 18 to provide a better fit to a part of an orthotic device 2, such as the heel area of a foot orthotic 2 in this example.

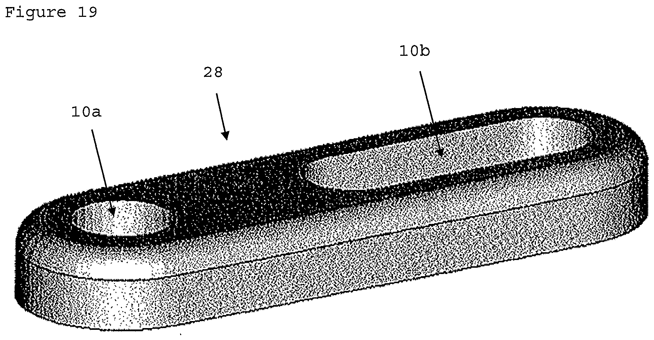

Referring to Figure 19, a stiffness adjusting member 6 is shown in the form of a connector 28 containing two recesses (10a, 10b) as second engaging means, wherein a first recess 10a is longer than a second recess 10b along the connection axis of the connector 28, to restrict bending of the part of the orthotic 2 onto which it is engaged to only the direction parallel to the axis of connection. This provides further possibilities for adjustment of the stiffness of the orthotic 2.

It will be appreciated by persons skilled in the art that the above embodiments have been described by way of example only and not in any limitative sense, and that various alterations and modifications are possible without departure from the scope of the invention as defined by the appended claims. For example, the protrusions 8, as well as being cylindrical, can be any suitable shape, such as half-spherical, triangular, rectangular or square etc.

## Claims

1. A support apparatus (2, 18, 20, 22) comprising:
a body (4) adapted to support a body part of a user; and
at least one stiffness adjusting member (6, 24, 26, 28) adapted to be removably mounted to said body (4) to adjust the stiffness of the apparatus (2, 18, 20, 22);
wherein said body (4) comprises a plurality of first engaging members (8) and at least one said stiffness adjusting member (6, 24, 26, 28) comprises a plurality of second engaging members (10) for removably engaging at least two of said first engaging members (8) to mount the stiffness adjusting member (6, 24, 26, 28) to the body (4) ;
**characterised in that** said first engaging members (8) are arranged to allow re-engagement of said stiffness adjusting member (6, 24, 26, 28) to another location on said body (4) and/or engagement of a plurality of said stiffness adjusting members (6, 24, 26, 28) at different respective locations on said body (4), wherein a number of said first engaging members (8) provided on said body (4) is greater than a number of said second engaging members (10) provided on said stiffness adjusting member (6, 24, 26, 28).

2. The apparatus according to claim 1, wherein said plurality of first engaging members (8) and/or said plurality of second engaging members (10) comprises a respective plurality of recesses and/or protrusions.

3. The apparatus according to claim 1 or claim 2, wherein:
said plurality of first engaging members (8) comprises at least one first recess and at least one first protrusion; and
said plurality of second engaging members (10) comprises at least one second recess and at least one second protrusion.

4. The apparatus according to any of the preceding claims, wherein said body (4) and/or said first engaging members (8) and/or said second engaging members (10) and/or at least one said stiffness adjusting member (6, 24, 26, 28) is manufactured by additive manufacturing.

5. The apparatus according to any one of the preceding claims, wherein the apparatus (2, 18, 20, 22) is an orthotic apparatus.

6. The apparatus according to any one of claims 1 to 5,
wherein the apparatus (2, 18, 20, 22) is a prosthetic apparatus (2, 18, 20, 22).

7. The apparatus according to any one of the preceding claims, wherein said first engaging members (8) are arranged to allow replacement of said stiffness adjusting member (6, 24, 26, 28) by a further stiffness adjusting member (6, 24, 26, 28) having a different configuration,

8. The apparatus claim 7, wherein said further stiffness adjusting member (6, 24, 26, 28) comprises a different number and/or arrangement of second engaging members (10) and/or is adapted to connect a different subset of said plurality of first engagement members (8).

9. The apparatus according to any one of the preceding claims, wherein said body (4) is shaped to a body part of a user.

10. The apparatus according to any one of the preceding claims, wherein a thickness of said stiffness adjusting member (6, 24, 28) is substantially uniform between said second engaging members (10).

11. The apparatus according to any one of claims 1 to 9,
wherein said stiffness adjusting member (26) is contoured to fit a part of the body (4).

12. A kit comprising an apparatus (2, 18, 20, 22) according to any one of the preceding claims and at least one further stiffness adjusting member (6, 24, 26, 28).

13. A method of altering the stiffness of a support apparatus (2, 18, 20, 22), the method comprising:
providing a support apparatus (2, 18, 20, 22) according to any one of claims 1 to 12;
mounting said at least one stiffness adjusting member (6, 24, 26, 28) to said body (4) by engaging at least two of said first engaging members (8) and at least two respective said second engaging members (10);
further comprising at least one of the following features:
re-engaging said stiffness adjusting member (6, 24, 26, 28) to a different location on said body (4); and/or
engaging a further said stiffness adjusting member (6, 24, 26, 28) at a different location on said body (4).

14. The method according to claim 13, further comprising:
applying tensile or compressive force to said body (4) prior to mounting at least one said stiffness adjusting member (6, 24, 26, 28) to said body (4).

15. The method according to claim 13 or claim 14, further comprising: replacing said stiffness adjusting member (6, 24, 26, 28) by a further stiffness adjusting member (6, 24, 26, 28) having a different configuration.

## Patentansprüche

1. Eine Trägervorrichtung (2, 18, 20, 22), umfassend:
einen Körper (4), eingerichtet um ein Körperteil eines Nutzers zu stützen; und
mindestens ein Element (6, 24, 26, 28) zum Einstellen der Steifigkeit, dazu eingerichtet, an dem besagten Körper (4) lösbar befestigt zu sein, um die Steifigkeit der Vorrichtung (2, 18, 20, 22) einzustellen;
wobei der besagte Körper (4) eine Vielzahl von ersten eingreifenden Elementen (8) umfasst, und mindestens ein besagtes Element (6, 24, 26, 28) zum Einstellen der Steifigkeit eine Vielzahl von zweiten eingreifenden Elementen (10) zum lösbaren Eingreifen in mindestens zwei der besagten ersten eingreifenden Elementen (8) umfasst, um das Element (6, 24, 26, 28) zum Einstellen der Steifigkeit an dem Körper (4) zu befestigen;
**dadurch gekennzeichnet, dass** die besagten ersten eingreifenden Elemente (8) angeordnet sind um einen Wiedereingriff des besagten Elements (6, 24, 26, 28) zum Einstellen der Steifigkeit an einem anderen Ort auf dem besagten Körper (4) zu ermöglichen und/oder einen Eingriff von einer Vielzahl der besagten Elemente (6, 24, 26, 28) zum Einstellen der Steifigkeit an verschiedenen entsprechenden Orten auf dem besagten Körper (4) zu ermöglichen, wobei eine Anzahl von besagten ersten eingreifenden Elementen (8), die auf dem besagten Körper (4) vorgesehen sind, größer ist, als eine Anzahl von besagten zweiten eingreifenden Elementen (10), die auf den besagten Elementen (6, 24, 26, 28) zum Einstellen der Steifigkeit vorgesehen sind.

2. Die Vorrichtung nach Anspruch 1, wobei die besagte Vielzahl von ersten eingreifenden Elementen (8) und/oder die besagte Vielzahl von zweiten eingreifenden Elementen (10) eine entsprechende Vielzahl von Ausnehmungen und/oder Vorsprüngen umfasst.

3. Die Vorrichtung nach Anspruch 1 oder Anspruch 2, wobei:
die besagte Vielzahl von ersten eingreifenden Elementen (8) mindestens eine erste Ausnehmung und mindestens einen ersten Vorsprung umfasst; und
die besagte Vielzahl von zweiten eingreifenden Elementen (10) mindestens eine zweite Ausnehmung und mindestens einen zweiten Vorsprung umfasst.

4. Die Vorrichtung nach einem der vorstehenden Ansprüche, wobei besagter Körper (4) und/oder besagte erste eingreifende Elemente (8) und/oder besagte zweite eingreifende Elemente (10) und/oder mindestens ein besagtes Element (6, 24, 26, 28) zum Einstellen der Steifigkeit durch additive Fertigung hergestellt ist.

5. Die Vorrichtung nach einem der vorstehenden Ansprüche, wobei die Vorrichtung (2, 18, 20, 22) eine orthetische Vorrichtung ist.

6. Die Vorrichtung nach einem der Ansprüche 1 bis 5, wobei die Vorrichtung (2, 18, 20, 22) eine prothetische Vorrichtung (2, 18, 20, 22) ist.

7. Die Vorrichtung nach einem der vorstehenden Ansprüche, wobei besagte erste eingreifende Elemente (8) angeordnet sind um einen Austausch des besagten Elements (6, 24, 26, 28) zum Einstellen der Steifigkeit durch ein weiteres Element (6, 24, 26, 28) zum Einstellen der Steifigkeit zu erlauben, welches eine andere Konfiguration hat.

8. Die Vorrichtung nach Anspruch 7, wobei besagtes weiteres Element (6, 24, 26, 28) zum Einstellen der Steifigkeit eine andere Anzahl und/oder Anordnung der zweiten eingreifenden Elemente (10) umfasst und/oder dazu eingerichtet ist, eine andere Teilmenge der besagten Vielzahl von ersten eingreifenden Elementen (8) zu verbinden.

9. Die Vorrichtung nach einem der vorstehenden Ansprüche, wobei besagter Körper (4) an ein Körperteil eines Nutzers angepasst ist.

10. Die Vorrichtung nach einem der vorstehenden Ansprüche, wobei eine Dicke des besagten Elements (6, 24, 28) zum Einstellen der Steifigkeit zwischen besagten zweiten eingreifenden Elementen (10) im Wesentlichen einheitlich ist.

11. Die Vorrichtung nach einem der Ansprüche 1 bis 9, wobei besagtes Element (26) zum Einstellen der Steifigkeit konturiert ist, um sich an einen Teil des Körpers (4) anzupassen.

12. Eine Ausrüstung, umfassend eine Vorrichtung (2, 18, 20, 22) nach einem der vorstehenden Ansprüche und mindestens ein weiteres Element (6, 24, 26, 28) zum Einstellen der Steifigkeit.

13. Ein Verfahren zum Ändern der Steifigkeit einer Trägervorrichtung (2, 18, 20, 22), wobei das Verfahren umfasst:
Bereitstellen einer Trägervorrichtung (2, 18, 20, 22) nach einem der Ansprüche 1 bis 12;
Befestigen von besagtem mindestens einem Element (6, 24, 26, 28) zum Einstellen der Steifigkeit an dem besagten Körper (4) durch Ineingriffbringen von mindestens zwei der besagten ersten eingreifenden Elemente (8) und mindestens zwei entsprechenden besagten zweiten eingreifenden Elementen (10);
ferner umfassend mindestens eines der folgenden Merkmale:
Wiederineingriffbringen des besagten Elements (6, 24, 26, 28) zum Einstellen der Steifigkeit an einem anderen Ort auf dem besagten Körper (4); und/oder Ineingriffbringen eines weiteren besagten Elements (6, 24, 26, 28) zum Einstellen der Steifigkeit an einem anderen Ort auf dem besagten Körper (4).

14. Das Verfahren nach Anspruch 13, ferner umfassend:
Anwenden von Zugkraft oder Druckkraft auf den besagten Körper (4) vor dem Befestigen von mindestens einem besagten Element (6, 24, 26, 28) zum Einstellen der Steifigkeit an dem besagten Körper (4).

15. Das Verfahren nach Anspruch 13 oder Anspruch 14, ferner umfassend:
Ersetzen des besagten Elements (6, 24, 26, 28) zum Einstellen der Steifigkeit durch ein weiteres Element (6, 24, 26, 28) zum Einstellen der Steifigkeit, welches eine andere Konfiguration hat.

## Revendications

1. Appareil de support (2, 18, 20, 22) comprenant :
un corps (4) adapté pour supporter une partie de corps d'un utilisateur ; et
au moins un élément de réglage de la rigidité (6, 24, 26, 28) adapté pour être monté de manière amovible sur ledit corps (4) afin de régler la rigidité de l'appareil (2, 18, 20, 22) ;
dans lequel ledit corps (4) comprend une pluralité de premiers éléments d'engagement (8) et au moins l'un desdits éléments de réglage de la rigidité (6, 24, 26, 28) comprend une pluralité de deuxièmes éléments d'engagement (10) pour s'engager de manière amovible avec au moins deux desdits premiers éléments d'engagement (8) pour monter l'élément de réglage de la rigidité (6, 24, 26, 28) sur le corps (4) ;
**caractérisé en ce que** lesdits premiers éléments d'engagement (8) sont agencés pour permettre un réengagement dudit élément de réglage de la rigidité (6, 24, 26, 28) à un autre emplacement sur ledit corps (4) et/ou un engagement d'une pluralité desdits éléments de réglage de la rigidité (6, 24, 26, 28) à différents emplacements respectifs sur ledit corps (4), dans lequel le nombre desdits premiers éléments d'engagement (8) prévus sur ledit corps (4) est supérieur au nombre desdits deuxièmes éléments d'engagement (10) prévus sur ledit élément de réglage de la rigidité (6, 24, 26, 28).

2. Appareil selon la revendication 1, dans lequel ladite pluralité de premiers éléments d'engagement (8) et/ou ladite pluralité de deuxièmes éléments d'engagement (10) comprennent une pluralité respective d'évidements et/ou de saillies.

3. Appareil selon la revendication 1 ou 2, dans lequel :
ladite pluralité de premiers éléments d'engagement (8) comprend au moins un premier évidement et au moins une première saillie ; et
ladite pluralité de deuxièmes éléments d'engagement (10) comprend au moins un deuxième évidement et au moins une deuxième saillie.

4. Appareil selon l'une des revendications précédentes, dans lequel ledit corps (4) et/ou lesdits premiers éléments d'engagement (8) et/ou lesdits deuxièmes éléments d'engagement (10) et/ou au moins l'un desdits éléments de réglage de la rigidité (6, 24, 26, 28) est/sont fabriqué(s) par fabrication additive.

5. Appareil selon l'une quelconque des revendications précédentes, dans lequel l'appareil (2, 18, 20, 22) est un appareil orthétique.

6. Appareil selon l'une quelconque des revendications 1 à 5, dans lequel l'appareil (2, 18, 20, 22) est un appareil prothétique (2, 18, 20, 22).

7. Appareil selon l'une quelconque des revendications précédentes, dans lequel lesdits premiers éléments d'engagement (8) sont agencés pour permettre le remplacement dudit élément de réglage de la rigidité (6, 24, 26, 28) par un élément supplémentaire de réglage de la rigidité (6, 24, 26, 28) ayant une configuration différente.

8. Appareil selon la revendication 7, dans lequel ledit élément supplémentaire de réglage de la rigidité (6, 24, 26, 28) comprend un nombre et/ou un agencement différent(s) de deuxièmes éléments d'engagement (10) et/ou est adapté pour connecter un sous-ensemble différent de ladite pluralité de premiers éléments d'engagement (8).

9. Appareil selon l'une quelconque des revendications précédentes, dans lequel ledit corps (4) a la forme d'une partie de corps d'un utilisateur.

10. Appareil selon l'une quelconque des revendications précédentes, dans lequel une épaisseur dudit élément de réglage de la rigidité (6, 24, 28) est essentiellement uniforme entre lesdits deuxièmes éléments d'engagement (10).

11. Appareil selon l'une quelconque des revendications 1 à 9, dans lequel ledit élément de réglage de la rigidité (26) est profilé pour s'adapter à une partie du corps (4).

12. Kit comprenant un appareil (2, 18, 20, 22) selon l'une quelconque des revendications précédentes et au moins un élément supplémentaire de réglage de la rigidité (6, 24, 26, 28).

13. Procédé de modification de la rigidité d'un appareil de support (2, 18, 20, 22), le procédé comprenant le fait :
de fournir un appareil de support (2, 18, 20, 22) selon l'une quelconque des revendications 1 à 12 ;
de monter ledit au moins un élément de réglage de la rigidité (6, 24, 26, 28) sur ledit corps (4) en engageant au moins deux desdits premiers éléments d'engagement (8) et au moins deux desdits deuxièmes éléments d'engagement respectifs (10) ;
comprenant en outre au moins l'une des caractéristiques suivantes consistant :
à réengager ledit élément de réglage de la rigidité (6, 24, 26, 28) à un emplacement différent sur ledit corps (4) ; et/ou
à engager ledit élément supplémentaire de réglage de la rigidité (6, 24, 26, 28) à un emplacement différent sur ledit corps (4).

14. Procédé selon la revendication 13, comprenant en outre le fait :
d'appliquer une force de traction ou de compression sur ledit corps (4) avant de monter au moins l'un desdits éléments de réglage de la rigidité (6, 24, 26, 28) sur ledit corps (4).

15. Procédé selon la revendication 13 ou 14, comprenant en outre le fait :
de remplacer ledit élément de réglage de la rigidité (6, 24, 26, 28) par un élément supplémentaire de réglage de la rigidité (6, 24, 26, 28) ayant une configuration différente.
